(19) Europäisches Patentamt / European Patent Office / Office européen des brevets

(11) EP 4 763 207 A1

(12) **EUROPEAN PATENT APPLICATION**
published in accordance with Art. 153(4) EPC

(43) Date of publication:
24.06.2026 Bulletin 2026/26

(21) Application number: 24853595.7

(22) Date of filing: 05.08.2024

(51) International Patent Classification (IPC):
A61K 31/4422 (2006.01)    A61K 9/00 (2006.01)
A61P 9/10 (2006.01)    A61P 25/00 (2006.01)

(52) Cooperative Patent Classification (CPC):
A61K 31/4422; A61K 9/0043; A61K 47/08;
A61K 47/14; A61K 47/24

(86) International application number:
PCT/CN2024/109725

(87) International publication number:
WO 2025/036183 (20.02.2025 Gazette 2025/08)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 17.08.2023 CN 202311045174

(71) Applicant: Nasal-Phyto (Shanghai)
Pharmaceutical
Technology Co., Ltd.
Shanghai 201203 (CN)

(72) Inventors:
• LIU, Jingyi
  Shanghai 201203 (CN)
• YOU, Qing
  Shanghai 201203 (CN)
• WANG, Chengwei
  Shanghai 201203 (CN)

(74) Representative: Verscht, Thomas Kurt Albert
Josephsburgstrasse 88 A
81673 München (DE)

(54) **NIMODIPINE NOSE-TO-BRAIN DELIVERY PREPARATION, PREPARATION METHOD THEREFOR AND USE THEREOF**

(57) Provided is a highly brain-targeted, safe and non-invasive nose-to-brain delivery preparation. The nose-to-brain delivery preparation comprises nimodipine, a good solvent, a diluent and an absorption enhancer, wherein the good solvent is diethylene glycol monoethyl ether, and the absorption enhancer is phospholipid. The nose-to-brain delivery preparation is prepared from a good solvent, a diluent, an absorption enhancer and other components in a specific proportion. By administrating the preparation by means of a specific superior nasal meatus administration technique, the active ingredient nimodipine is enriched in the cerebrospinal fluid and brain tissue, thus exerting a therapeutic effect; meanwhile, due to the fact that the proportion of nimodipine entering the bloodstream is reduced, cardiovascular toxicity and hepatotoxicity are reduced. In addition, the formula of the preparation does not irritate the local part of the nasal cavity, thereby improving the compliance of the patient.

FIG. 1

**Description**

**FIELD OF THE DISCLOSURE**

The present disclosure belongs to the field of pharmaceuticals, relates to a nose-to-brain delivery formulation of a drug, in particular relates to the nose-to-brain delivery formulation with a high safety and a high brain-targeted property, and specifically relates to the nose-to-brain delivery formulation of nimodipine with the high safety and the high brain-targeted property.

**BACKGROUND OF THE DISCLOSURE**

Nimodipine (NM) is the second generation of dihydropyridine calcium channel antagonist, which can inhibit calcium influx in cerebral vascular smooth muscle cells and cerebral neuronal cells, the drug acts preferentially on small arteries, which can selectively dilate cerebral blood vessels, increase cerebral blood flow, protect ischemic neuronal cells, promote memory and facilitate intellectual recovery, and have both neuro-psychopharmacological effects. It is clinically used in the treatment of cerebral ischemic diseases, cerebral vasospasm induced by subarachnoid hemorrhage, sudden deafness, and other diseases, and achieves favorable therapeutic effects. However, the water solubility of nimodipine is extremely poor, the oral first-pass effect is strong, the bioavailability is low, and the clinically used injections employ a large amount of ethanol as a solvent, resulting in high irritation. Nimodipine also has cardiovascular toxicity and hepatotoxicity. In addition, the blood-brain barrier is the most important bottleneck for the therapeutic effects of the drug in central diseases., nimodipine is not effective in crossing the blood-brain barrier, resulting in a low brain delivery efficiency, and thus failing to maximize the therapeutic effects on the brain diseases.

Nose-to-brain delivery system is mainly for upper nasal meatus drug delivery, as a non-invasive drug delivery, it has received wide attention in recent years. The mucosa of the olfactory region of the upper nasal meatus has many microscopic villi, which can greatly increase the absorption effective area of the drug, the nose-to-brain delivery has the following advantages: ① Convenient drug administration, and it is desirable for patients to self-administer the drug; ② Rapid absorption of the drug and fast onset of action; (3) Avoidance of hepatic first-pass effect; and ④ Increase in the delivery of the drug to the brain. Therefore, nasal drug delivery is expected to increase the drug concentration in the brain, enhance the therapeutic effects of the drug and reduce the toxic and side effects.

In clinical practice, it has been found that although the olfactory nerve pathway in the olfactory region of the upper nasal cavity is a noninvasive and convenient route for central drug delivery, drug delivery to the olfactory region of the upper nasal meatus is limited by many physiological factors. Conventional drug delivery devices cannot effectively deliver the drug to the nasal olfactory region, thus rendering the goal of nose-to-brain drug delivery unattainable. Especially with the help of animal models for the indirect study of human diseases, small animals as the most common and conventional mammalian model carriers, however, many devices and apparatuses are unsuitable for use in animals of smaller size owing to their small sizes, thus posing a considerable obstacle to the establishment of animal models. For delivering the drug to upper nasal cavities in small animals, the devices disclosed in patent applications CN202021777601.0 and CN202010148279.3 of Applicant enable the drug delivery to the upper nasal meatus in small animals such as mice and a ensure stable administration under an anesthesia state and a constant speed, guaranteeing parallelism of preclinical studies; and a nasal cavity of rodents features a dense distribution of olfactory nerves, it is divided into an olfactory region and a respiratory region in primates, for clinical trials in humans, more precise administration techniques of the upper nasal meatus are required.

The nose-to-brain delivery formulation of nimodipine are administered via the upper nasal meatus, so a drug formulation should be prepared as the nose-to-brain delivery formulation characterized by high concentration, absence of nasal toxicity or irritation, compatibility with the nasal pH environment, and ability of transmembrane absorption into the brain, thus decreasing content of nimodipine in peripheral blood while decreasing cardiactoxicity and hepatictoxicity.

As nimodipine has a poor solubility, alcohol-based formulations are commonly employed for solubilization in clinical practice. Currently, studies on the formulation of nimodipine have focused on reducing the amount of ethanol, yet failed to decrease cardiotoxicity or hepatotoxicity.

Currently, commercially available Nimotop injection contains up to 20% ethanol, which is liable to cause injection-related irritation and thrombophlebitis.

Patent application CN201811046629.4 discloses nimodipine nanoemulsion injections. The formulations no longer use ethanol, thus reducing the occurrence probability of irritation and thrombophlebitis under the injection route. However, they have a low brain delivery, a high blood level and a high risk of cardiotoxicity and hepatotoxicity.

Patent application CN200510023273.9 discloses nimodipine gels for transnasal administration. However, these gels are applied to the lower nasal meatus, and the improvement is still to increase the amount of the drug in the bloodstream, and when the formulations in this patent application are applied to the upper nasal meatus, they have a very low permeability coefficient have difficult to achieve the transnasal brain-targeted delivery by adopting Franz diffusion cell model. As nimodipine works by entering the brain or cerebrospinal fluid, these formulations do not improve the therapeutic effects of the drug, and the risk of cardiotoxicity and hepatotoxicity remains high, due to the high content of the drug persisting in the bloodstream.

In addition, current studies of nasal mucosal delivery systems have found that the formulations can cause irritation to the nasal mucosa, primarily presenting as ciliotoxic effects; including the effects of the drug and excipients on ciliary activity. Literature reports indicate that nimodipine nasal cavity formulations contain the surfactant Labrasol (Role of mucoadhesive polymers in enhancing delivery of nimodipine microemulsion to brain via intranasal route, Rudree Pathak et al., Acta Pharmaceutica Sinica B, 2014; 4(2): 151-160). However, the applicant has verified that the formulations cannot be developed into the drug due to their high ciliary toxicity, and their concentration can only reach 7.5mg/mL, thus limiting their application in pharmaceuticals.

Therefore, how to ensure that nimodipine have both good nasal mucosal absorption performance and nasal cavity safety, as well as having high brain delivery and a high brain-to-blood ratio, is the key to whether nimodipine can be developed as a nasal upper-nasal-meatus drug delivery formulation.

## BRIEF SUMMARY OF THE DISCLOSURE

Aiming at the technical problems in the existing techniques that the nimodipine has a poor brain penetration rate, and the drug needs to pass the blood-brain barrier to enter the brain after being administered by the mucous membrane of the lower nasal meatus and intravenously, which results in a low concentration of the drug in the brain, with nimodipine in the peripheral circulatory system easily inducing the side effects of cardiovascular toxicity and hepatotoxicity, the present disclosure provides a reservoir-type nose-to-brain delivery formulation of nimodipine of high cerebrospinal fluid/olfactory bulb/cortical brain tissue concentration, free from local irritation in nasal cavity, capable of stably existing in the long term, entering the brain tissues directly by transmitting through the mucosa of the olfactory region of the upper nasal meatus, capable of being absorbed smoothly, and having a high brain-to-blood ratio and a high cerebrospinal fluid-to-blood ratio.

According to a first aspect of the present disclosure, the present disclosure provides a nose-to-brain delivery formulation of nimodipine, which comprises the nimodipine, a good solvent, a diluent and an absorption enhancer, wherein the good solvent is diethylene glycol monoethyl ether, and the absorption enhancer is one or more of phospholipid, n-dodecyl-β-D-maltoside (DDM), cyclopentadecanolide, polyoxyl 15 hydroxystearate and oleoyl polyoxyl-6 glycerides, preferably, the absorption enhancer is one of the phospholipid, the cyclopentadecanolide, and most preferably is the phospholipid.

The diluent of the nose-to-brain delivery formulation of the nimodipine of the present disclosure can be one or more selected from isopropyl myristate, ethyl oleate, medium chain triglycerides, castor oil, soybean oil, sesame oil, oleic acid, vitamin E, glyceryl monolinoleate, propylene glycol laurate or propylene glycol dicaprylate/dicaprate, and preferably, the diluent comprises one or a combination of multiple of the ethyl oleate, the isopropyl myristate and the propylene glycol dicaprylate/dicaprate.

In the nose-to-brain delivery formulation of the nimodipine of the present disclosure, the phospholipid can be lecithin or soya lecithin; and further, a model number of the lecithin can be any model number, for example, which can be lecithin E80 or lecithin PL100M, and a model number of the soya lecithin can be any model number, for example, which can be soya lecithin S75 or soya lecithin S100.

In the nose-to-brain delivery formulation of the nimodipine of the present disclosure, the nimodipine is 4-50 parts by weight, preferably, 5-40 parts by weight, for example, which can be 5, 10, 15, 20, 30, 40 parts by weight, most preferably, which is 15 parts by weight.

In the nose-to-brain delivery formulation of the nimodipine of the present disclosure, the good solvent is 5-300 parts by weight, preferably, which is 40-200 parts by weight, for example, which can be 40, 50, 80, 100, 120, 150, 160, 200 parts by weight.

In the nose-to-brain delivery formulation of the nimodipine of the present disclosure, the diluent is 500-900 parts by weight, preferably, which is 700-850 parts by weight, for example, which can be 725, 750, 785, 805, 825, 850 parts by weight, most preferably, which is 785 parts by weight.

In the nose-to-brain delivery formulation of the nimodipine of the present disclosure, the absorption enhancer is 20-200 parts by weight, preferably, which is 40-160 parts by weight, which can be 50 parts by weight, 60 parts by weight, 80 parts by weight, 100 parts by weight, 120 parts by weight, 150 parts by weight, 160 parts by weight, most preferably, which is 100 parts by weight.

The nose-to-brain delivery formulation of the nimodipine of the present disclosure is a solution.

In some embodiments of the present disclosure, the nose-to-brain delivery formulation of the nimodipine comprises the aforementioned nimodipine, the aforementioned good solvent, the aforementioned diluent, the aforementioned absorption enhancer.

In some embodiments of the present disclsoure, the nose-to-brain delivery formulation of the nimodipine comprises 5-40 parts by weight of the nimodipine, 40-200 parts by weight of the diethylene glycol monoethyl ether, 40-160 parts by weight of the phospholipid; 700-850 parts by weight of the isopropyl myristate, preferably, the phospholipid is the lecithin or the soya lecithin, wherein a model number of the lecithin can be any model number, for example, which can be lecithin E80 or lecithin PL100M, and a model number of the soya lecithin can be any model number, for example, which can be soya lecithin S75 or soya lecithin S100.

In some embodiments of the present disclsoure, the nose-to-brain delivery formulation of the nimodipine comprises 15 parts by weight of the nimodipine, 100 parts by weight of the diethylene glycol monoethyl ether, 100 parts by weight of the phospholipid, 785 parts by weight of the isopropyl myristate, preferably, the phospholipid is the lecithin or the soya lecithin, wherein a model number of the lecithin can be any model number, for example, which can be lecithin E80 or lecithin PL100M, and a model number of the soya lecithin can be any model number, for example, which can be soya lecithin S75 or soya lecithin S100.

In some embodiments of the present disclsoure, the nose-to-brain delivery formulation of the nimodipine comprises 5-40 parts by weight of the nimodipine, 40-200 parts by weight of the diethylene glycol monoethyl ether, 40-160 parts by weight of the phospholipid, 700-850 parts by weight of the propylene glycol dicaprylate/dicaprate, preferably, the phospholipid is lecithin or soya lecithin, wherein a model number of the lecithin can be any model number, for example, which can be lecithin E80 or lecithin PL100M, and a model number of the soya lecithin can be any model number, for example, which can be soya lecithin S75 or soya lecithin S100.

In some embodiments of the present disclsoure, the nose-to-brain delivery formulation of the nimodipine comprises 15 parts by weight of the nimodipine, 100 parts by weight of the diethylene glycol monoethyl ether, 100 parts by weight of the lecithin, 785 parts by weight of the propylene glycol dicaprylate/dicaprate, wherein a model number of the lecithin can be any model number, for example, which can be lecithin E80 or lecithin PL100M.

In some embodiments of the present disclsoure, the nose-to-brain delivery formulation of the nimodipine comprises 5-40 parts by weight of the nimodipine, 40-200 parts by weight of the diethylene glycol monoethyl ether, 40-160 parts by weight of the phospholipid, 700-850 parts by weight of the ethyl oleate, preferably, the phospholipid is lecithin or soybean lecithin, wherein a model number of the lecithin can be any model number, for example, which can be lecithin E80 or lecithin PL100M, a model number of the soya lecithin can be any model number, for example, which can be soya lecithin S75 or soya lecithin S100.

In some embodiments of the present disclsoure, the nose-to-brain delivery formulation of the nimodipine comprises 5 parts by weight of the nimodipine, 100 parts by weight of the diethylene glycol monoethyl ether, 100 parts by weight of the lecithin, 795 parts by weight of the ethyl oleate, wherein the lecithin can be any model number, lecithin E80 or lecithin PL100M.

In some embodiments of the present disclsoure, the nose-to-brain delivery formulation of the nimodipine comprises 15 parts by weight of the nimodipine, 60 parts by weight of the diethylene glycol monoethyl ether, 100 parts by weight of the lecithin, 825 parts by weight of the ethyl oleate, wherein the lecithin can be any model number, lecithin E80 or lecithin PL100M.

In some embodiments of the present disclsoure, the nose-to-brain delivery formulation of the nimodipine comprises 15 parts by weight of the nimodipine, 120 parts by weight of the diethylene glycol monoethyl ether, 100 parts by weight of the lecithin, 765 parts by weight of the ethyl oleate, wherein the lecithin can be any model number, lecithin E80 or lecithin PL100M.

In some embodiments of the present disclsoure, the nose-to-brain delivery formulation of the nimodipine comprises 15 parts by weight of the nimodipine, 100 parts by weight of the diethylene glycol monoethyl ether, 100 parts by weight of the phospholipid, 785 parts by weight of the ethyl oleate, the phospholipid is lecithin or soya lecithin, wherein a model number of the lecithin can be any model number, for example, which can be lecithin E80 or lecithin PL100M, and a model number of the soya lecithin can be any model number, for example, which can be soya lecithin S75 or soya lecithin S100.

In some embodiments of the present disclsoure, the nose-to-brain delivery formulation of the nimodipine comprises 15 parts by weight of the nimodipine, 100 parts by weight of the diethylene glycol monoethyl ether, 60 parts by weight of the phospholipid, 825 parts by weight of the ethyl oleate, the phospholipid is lecithin or soya lecithin, wherein a model number of the lecithin can be any model number, for example, which can be lecithin E80 or lecithin PL100M, and a model number of the soya lecithin can be any model number, for example, which can be soya lecithin S75 or soya lecithin S100.

In some embodiments of the present disclsoure, the nose-to-brain delivery formulation of the nimodipine comprises 15 parts by weight of the nimodipine, 100 parts by weight of the diethylene glycol monoethyl ether, 120 parts by weight of the phospholipid, 765 parts by weight of the ethyl oleate, the phospholipid is lecithin or soya lecithin, wherein a model number of the lecithin can be any model number, for example, which can be lecithin E80 or lecithin PL100M, and a model number of the soya lecithin can be any model number, for example, which can be soya lecithin S75 or soya lecithin S100.

In some embodiments of the present disclsoure, the nose-to-brain delivery formulation of the nimodipine comprises the aforementioned nimodipine, the aforementioned good solvent, the aforementioned diluent, the aforementioned absorption enhancer; preferably, which further contains aromatizer, the aromatizer is preferably grape essence; preferably, a content of essence is 0-50 parts by weight; and more preferbaly 1-10 parts by weight; for example, 2.5 parts by weight.

In some embodiments of the present disclsoure, the nose-to-brain delivery formulation of the nimodipine of the present disclosure with a high concenction in an olfactory region and a brain can act as a drug reservoir, it can maintain a more stable concentration in cerebrospinal fluid for a long time, thereby exerting a prolonged therapeutic effect.

In a particularly preferred embodiment, the nose-to-brain delivery formulation of the nimodipine of the present disclosure consists of the aforementioned nimodipine, the aforementioned good solvent, the aforementioned diluent and the aforementioned absorption enhancer.

In another particularly preferred embodiment, the nose-to-brain delivery formulation of nimodipine of the present disclosure is administrated by Intelligent Integrated Nasal Drug Delivery System for Small Animals (RA-IDDS®) developed by the company of Applicant, as disclosed in CN202021777601.0 and CN202010148279.3.

According to a second aspect of the present disclosure, the present disclosure provides a method for preparing the nose-to-brain delivery formulation of the nimodipine, adding the nimodipine and the aforementioned good solvent to a glass vial, and preferably, dissolving by sonication; and adding the aforementioned absorption enhancer, and preferably, dissolving by sonication and shaking; and optionally, further adding the aromatizer, and finally, obtaining by adding the aforemnetioned diluent to a total mass of each composition, and preferably, obtaining via mixing by rotation.

According to a third aspect of the present disclosure, the present disclosure provides the nose-to-brain delivery formulation of the nimodipine prepared by the aforementioned method of the nose-to-brain delivery formulation of the nimodipine.

According to a fourth aspect of the present disclosure, the present disclosure discloses a method for preventing or treating cerebral ischemic diseases, cerebral vasospasm induced by subarachnoid hemorrhage, sudden deafness of a patient, which comprises a step of administrating the nose-to-brain delivery formulation of the nimodipine to the patient with a therapeutic effective amount.

According to a fifth aspect of the present disclosure, the present disclosure provide a use of the nose-to-brain delivery formulation of the nimodipine in treating the cerebral ischemic diseases, the cerebral vasospasm induced by the subarachnoid hemorrhage, the sudden deafness via a nose-to-brain delivey. Specifically, the present disclosure provide a use of the nose-to-brain delivery formulation of the nimodipine in a preparation of drug for treating the cerebral ischemic diseases, the cerebral vasospasm induced by the subarachnoid hemorrhage, the sudden deafness.

In accordance with common knowledge in the art, the aforementioned preferred conditions can be combined arbitrarily to obtain various preferred embodiments of the present disclosure.

The advantages of the present disclosure are as follows:

The nose-to-brain delivery formulation of the nimodipine in the present disclosure, by selecting suitable ones of the diluent, the good solvent, the absorption enhancer and a reasonable ratio thereof,

1. The solubility of the nimodipine is increased from 0.002 mg/mL to 13.5 mg/mL by more than 6000 times;

No nasal cavity irritation and cilia toxicity, no damage to the nasal mucosa, high safety;
Avoiding a phenomenon of abnormally high brain intake caused by direct drug entry into the brain, which results from the destruction of nasal mucosa due to the nasal cavity irritation;
Improving an ability of transmembrane absorption of the nimodipine, permeability coefficient can reach $2\text{-}10*10^{-7}$ (cm/s), the higher the permeability coefficient, the easier the access to the olfactory bulb and the brain by passing through the mucosa of the nasal olfactory region and ;
The nose-to-brain delivery formulation of the nimodipine in the present disclosure can form a drug reservoir and exert a therapeutic effect for a long time through reasonable compounding, especially through reasonable selection of diluents with sustained-release effects, which is conducive to slow and smooth release of the drug nimodipine in the brain;
The nose-to-brain delivery formulation of the nimodipine of the present disclosure can directly enter the brain by bypassing the blood-brain barrier and exert the therapeutic effect directly, and the DTP (direct transfer percentage) to the cerebrospinal fluid and the olfactory bulb is close to 90%.

Therefore, the nose-to-brain delivery formulation of the nimodipine in the present disclosure can directly enter the cerebrospinal fluid and the olfactory region without passing through the blood-brain barrier, and its DTP (direct transfer percentage) to the cerebrospinal fluid and the olfactory bulb is close to 90%; and the high concentration of the nimodipine in the olfactory region and the brain can serve as the drug reservoir and can be maintained at a more stable concentration for a long time in the cerebrospinal fluid so as to exert the prolonged therapeutic effect. At the same time, the presently disclosed formulation improves solubility of the nimodipine through reasonable dosage compounding, and has a higher permeability coefficient, so that the drug can reach the brain tissues at a higher concentration, and can maintain a more stable concentration for a long time in the brain so as to exert the prolonged therapeutic effect. The nose-to-brain delivery formulation of the nimodipine in the present disclosure is non-irritating to the nasal cavity, non-toxic to the nasal cilia, and it improves compliance of the patient while preventing a phenomenon such as abnormally high concentrations in the brain caused by direct drug brain intake due to the destruction of the nasal mucosa by the formulation, this provides a new and effective way of treating neurological diseases, and has a good prospect for industrial application.

## BRIEF DESCRIPTION OF THE DRAWINGS

Other features, objects and advantages of the present disclosure will become more apparent from the detailed description of the non-limiting embodiments with reference to the accompanying drawings:

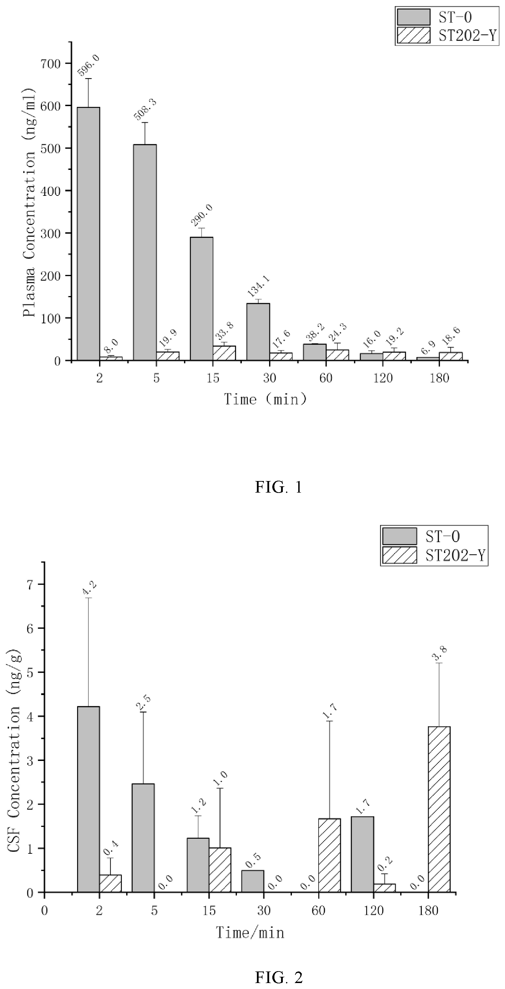

FIG. 1 is a graph of a comparison of drug concentrations in plasma after nose-to-brain delivery formulations of formulations 5-8 of Embodiment 5 of the present disclosure are administrated to the olfactory region of the upper nasal meatus of mice and after commercially available Nimotop® injection is injected intravenously into mice in Pharmacologic Embodiment 1.
FIG. 2 is a graph of a comparison of drug concentrations in cerebrospinal fluid after nose-to-brain delivery formulations of formulations 5-8 of Embodiment 5 of the present disclosure are administrated to the olfactory region of the upper nasal meatus of mice and after commercially available Nimotop® injection is injected intravenously into mice in pharmacological embodiment 1.

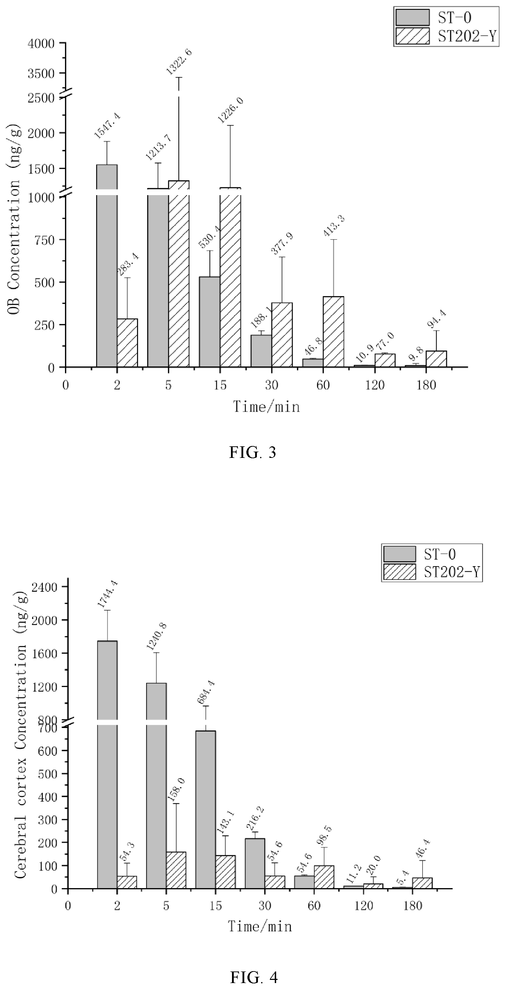

FIG. 3 is a graph of a comparison of drug concentrations in olfactory bulb after nose-to-brain delivery formulation of formulations 5-8 of Embodiment 5 of the present disclosure are adminstarted to the olfactory region of the upper nasal meatus of mice and after commercially available Nimotop® injection is injected intravenously into mice in pharmacological Embodiment 1.
FIG. 4 is a graph of a comparison of drug concentrations in cortical brain tissues after nose-to-brain delivery formulations of formulations 5-8 of Embodiment 5 of the present disclosure are administrated to the olfactory region of the upper nasal meatus of mice and after commercially available Nimotop® injection is injected intravenously into mice in pharmacological Embodiment 1.

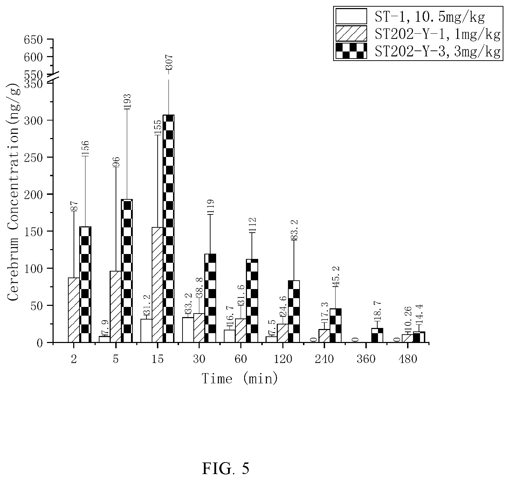

FIG. 5 is a graph of a comparison of drug concentrations in cerebrum after nose-to-brain delivery formulations ST202-Y-1 and ST202-Y-3 of the present disclosure are administrated to the olfactory region of the upper nasal meatus of mice and after commercially available Nimotop® tablet is dissolved and then administrated intragastrically into mice in pharmacological Embodiment 2.

## DETAILED DESCRIPTION OF THE EMBODIMENTS

The determination method of the present disclosure will be further described below in detail in combination with specific embodiments. It should be understood that the following embodiments are only exemplary to illustrate and explain the present disclosure, and should not be construed as a limitation of the scope of protection of the present disclosure. Any technology falls within the scope of the present disclosure intended to be protected provided that it is realized on the basis of the aforementioned contents of the present disclosure.

**Term Definition:**

In the present disclosure:

Transcutol P is diethylene glycol monoethyl ether;
Labrafil®M1944CS is oleoyl polyoxyl-6 glycerides;
HS15 is polyoxyl 15 hydroxystearate;
E80 is lecithin E80, and is a model number of lecithin;
PL100M is lecithin PL100M, and is a model number of lecithin;
S75 is soya lecithin S75, and is a model number of soya lecithin;
S100 is soya lecithin S100, and is a model number of soya lecithin;
DDM is n-dodecyl-$\beta$-D-maltoside;
CMC-Na is sodium carboxymethylcellulose.

Nose-to-brain delivery: drugs are delivered directly or indirectly into cerebrospinal fluid and brain tissues through a specific upper nasal meatus delivery method, and diffuse to exert effects in the brain.
Unless otherwise noted, the raw materials and the reagents used in the following embodiments are commercially available or can be prepared by known methods.

In the present disclosure, a determination method of a permeability coefficient adopts a method known to those of skill in the art: a Franz diffusion cell is used to simulate a process of drug absorption via a nasal mucosa. A drug solution is diffused through a porcine nasal mucosa via a supply pool to a receiving pool, and a drug solution in the receiving pool is collected at different time points, concentrations are measured, and an apparent permeability coefficient of the drug absorbed via the nasal mucosa is calculated.

In the present disclosure, nasal local irritation testing is determined using a model of ciliatoxicity, which is well known to those of skill in the art: toad palatal cilium is widely used as a commonly used model to simulate human nasal cilia [1]. A palatal mucosa is separated 60 minutes after administration by dropping the test drug onto the mucosal surface followed by washing, and a sustained movement of mucosal cilia is observed under a light microscope, its effect on the cilia is analyzed by using saline as a control [2], a cilia movement is observed, and a time required from a beginning of drug administration to a complete cessation of cilia movement is recorded, i.e., a ciliary sustained movement time. A relative percentage of the ciliary sustained movement time is obtained by dividing the ciliary sustained movement time of the palatal mucosa of the toads in a drug administration group by the ciliary sustained movement time of the palatal mucosa of the toads in a negative control group, the higher the percentage, the lower the toxicity to the cilia of the formulation, and the higher the safety of the formulation. The relative percentage of the ciliary sustained movement time being greater than 85% is a safety threshold of the formulation. ([1] Jiang, Xinguo, Cui, Jingbin. Toxicity of drugs on nasal mucocilia and the method of its evaluation [J]. Acta Pharmaceutica Sinica, 1995, 30:848-53. [2] Li, Xinfang, Li, Xiangui, Ma, Zhiqiang, et al. An in situgel system for nasal delivery of menthol:preparation and safety evaluation [J]. Journal of Pharmaceutical Practice, 2017, 3(4):321-4.)

It should be understood by those of skill in the art that in order to increase compliance of subjects to the nose-to-brain delivery formulation, aromatizer such as grape essence can also be added to adjust a taste of the nose-to-brain delivery formulation, and accordingly, an amount of diluent is reduced accordingly with an addition of the aromatizer, e.g., an addition of 2.5 parts by weight of the grape essence, 2.5 parts by weight of the diluent is reduced accordingly, this does not constitute a limitation of the nose-to-brain delivery formulation of the present disclosure.

Embodiment 1:

A composition of each formulation in a formulated amount is shown in Table 1 and is prepared at room temperature in the following manner, i.e., a formulated amount of nimodipine and a corresponding good solvent are weighed into a glass vial and dissolved by sonication; a corresponding amount of an absorption enhancer is then added according to specific components of each composition and is dissolved by shaking and sonication; the formulated amount of the diluent is finally added until a total mass of each composition reaches 1 g and mixed by rotation for 30 minutes to be dispersed until homogeneous to obtain the following composition; and permeability coefficients are tested.

Table 1

| Formulation number | | Embodiment 1-1 | Embodiment 1-2 |
|---|---|---|---|
| Nimodipine (mg) | | 15 | 15 |
| Good solvent | Transcutol P (mg) | 100 | 100 |
| Absorption enhancer | DDM (mg) | \ | 2 |
| Castor oil (mg) | | 885 | 883 |
| Permeability coefficient (cm/s) $10^{-7}$ | $2.21 \pm 0.16$ | $2.26 \pm 0.45$ | |

Experimental conclusion: the nose-to-brain delivery formulations of the nimodipine, using Transcutol P as the good solvent and castor oil as the diluent, the permeability coefficients are all greater than 2; and an addition of DDM has little effect on the permeability coefficients.

Embodiment 2:

A composition of each formulation in a formulated amount is shown in Table 2 and is prepared in accordance with the method described in embodiment 1, i.e., a formulated amount of nimodipine and Transcutol P are weighed into a glass vial and dissolved by sonication; a corresponding amount of an absorption enhancer is then added according to specific components of each composition and is dissolved by shaking and sonication; and the formulated amount of the diluent is finally added until a total mass of each composition reaches 1 g and mixed by rotation for 30 minutes to be dispersed until homogeneous to obtain the following composition, and permeability coefficients are tested.

Table 2

| Formulation number | | Embodiment 2-1 | Embodiment 2-2 | Embodiment 2-3 | Embodiment 2-4 | Embodiment 2-5 | Embodiment 2-6 |
|---|---|---|---|---|---|---|---|
| Nimodipine (mg) | | 15 | 15 | 15 | 15 | 15 | 15 |
| Transcutol P (mg) | | 100 | 100 | 100 | 100 | 100 | 100 |
| Absorption enhancer | Species | Cyclopenta decanolide | Cyclopenta decanolide | Cyclopenta decanolide | Lecithin | Lecithin | HS15 |
| | Dose (mg) | 40 | 40 | 40 | 40 | 40 | 1 |
| Diluent | Species | Castor oil | Isopropyl myristate | Oleic acid | Oleic acid | Isopropyl myristate | Oleic acid |
| | Dose (mg) | 845 | 845 | 845 | 845 | 845 | 884 |
| Permeability coefficient (cm/s) $10^{-7}$ | | 2.38 ± 1.12 | 3.03 ± 0.96 | 1.99 ± 0.48 | 3.34 ± 0.85 | 3.99 ± 1.20 | 2.54 ± 0.44 |

Experimental conclusion: lecithin and cyclopentadecanolide are selected as the absorption enhancer, which helps to increase the permeability coefficients of the nasal mucosa; adsorption of the sample of isopropyl myristate is the best among the different diluents; and the permeability coefficients reach a critical value when 4% cyclopentadecanolide is selected as the absorption enhancer and oleic acid is selected as the diluent.

Embodiment 3:

This embodiment compares different weight parts of lecithin, cyclopentadecanolide, lecithin plus cyclopentadecanolide and lecithin plus HS15 used as an absorption enhancer on the permeability coefficients of the nasal mucosa of the nose-to-brain delivery formulation of nilmodipine of the present disclosure.

A composition of each formulation in a formulated amount is shown in Table 3 and 4 and is prepared at room temperature in the following manner, i.e., a formulated amount of nimodipine and a corresponding good solvent are weighed into a glass vial and dissolved by sonication; a corresponding amount of the absorption enhancer is then added according to specific components of each composition and is dissolved by shaking and sonication; the formulated amount of the diluent is finally added until a total mass of each composition reaches 1 g and mixed by rotation for 30 minutes to be dispersed until homogeneous to obtain the following composition; and permeability coefficients are tested.

Table 3

| Composition number | | Composition 3-1 | Composition 3-2 | Composition 3-3 | Composition 3-4 |
|---|---|---|---|---|---|
| Nimodipine (mg) | | 15 | 15 | 15 | 15 |
| Good solvent | Transcutol P (mg) | 100 | 100 | 100 | 100 |
| Absorption enhancer | Species | Lecithin | | Cyclopentadecanolide | |
| | Dose (mg) | 100 | 160 | 100 | 160 |
| Diluent | Isopropyl myristate (mg) | 785 | 725 | 785 | 725 |
| Permeability coefficient (cm/s)$10^{-7}$ | | 5.02 ± 0.94 | 3.18 ± 1.14 | 3.87 ± 0.75 | 3.06 ± 0.86 |

Table 4

| Composition number | Composition 3-5 | Composition 3-6 | Composition 3-7 | Composition 3-8 |
|---|---|---|---|---|
| Nimodipine (mg) | 15 | 15 | 15 | 15 |

(continued)

| Composition number | | Composition 3-5 | Composition 3-6 | Composition 3-7 | Composition 3-8 |
|---|---|---|---|---|---|
| Good solvent | Transcutol P (mg) | 100 | 100 | 100 | 100 |
| Absorption enhancer | Species | Lecithin plus cyclopentadecanolide | | Lecithin plus HS15 | |
| | Dose (mg) | 100+40 | 100+100 | 100+1 | 100+2.5 |
| Isopropyl myristate (mg) | | 745 | 685 | 784 | 782.5 |
| Permeability coefficient (cm/s) $10^{-7}$ | | $3.32 \pm 1.08$ | $2.57 \pm 0.75$ | $4.30 \pm 0.89$ | $4.17 \pm 0.60$ |

Experimental conclusions: firstly, for an amount of the absorption enhancer, it is found that the highest value of the permeability coefficients of a corresponding porcine nasal mucosa is obtained when 10% lecithin or cyclopentadecanolide are used (*see* composition 3-1 and composition 3-2; and composition 3-3 and composition 3-4 in Table 3, respectively). Secondly, for species of the absorption enhancer, it is found that the highest value of the permeability coefficients of the corresponding porcine nasal mucosa is obtained when 10% lecithin is used (*see* composition 3-1 in Table 3). However, it is found that the use of lecithin in combination with cyclopentadecanolide and HS15 as the absorption enhancer both led to a decrease in the permeability coefficients (*see* compositions 3-5 to 3-8 in Table 4, respectively), and that the permeability coefficients decrease with an increase in an amount of cyclopentadecanolide and HS15 (*see* compositions 3-5 to 3-8 in Table 4).

Accordingly, when phospholipid is used alone as the absorption enhancer, the nose-to-brain delivery formulation of the nimodipine of this embodiment has an optimal permeability coefficient. An amount of phospholipid can be selected as 20-200 parts by weight, preferably 40-160 parts by weight.

Embodiment 4:

This embodiment compares effects of using lecithin E80, lecithin PL100M, soya lecithin S75 and soya lecithin S100 as absorption enhancers and the using isopropyl myristate, propylene glycol dicaprylate/dicaprate, ethyl oleate and propylene glycol laurate as diluents on appearance, nasal cavity local irritation, and permeability coefficients of nasal mucosa of the nose-to-brain delivery formulation of nimodipine of the present disclosure.

A preparation method of he compositions shown in Tables 5 and 6 below is similar to that of Embodiment 1, a formulated amount of 15 mg of the nimodipine and 100 mg of a good solvent Transcutol P is weighted into a glass vial and dissolved by sonication (for about 7 minutes), phospholipid corresponding to different compositions is added, and dissolved by shaking and sonication (for about 5 minutes); and finally, a diluent corresponding to the different compositions is added until a total mass is 1 g, mixing by rotation for 30 minutes to to be dispersed until homogeneous; the following composition is obtained.

Table 5

| Composition number | | Composition 4-1 | Composition 4-2 | Composition 4-3 |
|---|---|---|---|---|
| Nimodipine (mg) | | 15 | 15 | 15 |
| Good solvent | Transcutol P (mg) | 100 | 100 | 100 |
| Absorption enhancer, phospholipid | Species | E80 | PL100M | S75 |
| | Dose (mg) | 100 | 100 | 100 |
| Diluent | Species | Isopropyl myristate | | |
| | Dose (mg) | 785 | 785 | 785 |
| Appearance (room temperature) | | Light yellow transparent | yellow transparent | Orange transparent |
| Permeability coefficient (cm/s) $10^{-7}$ | | $5.6 \pm 0.5$ | $8.5 \pm 1.4$ | $7.0 \pm 2.2$ |
| Nasal cavity local irritation | | Reach the standard | Reach the standard | Reach the standard |

Table 6

| Composition number | | Composition 4-4 | Composition 4-5 | Composition 4-6 |
|---|---|---|---|---|
| Nimodipine (mg) | | 15 | 15 | 15 |
| Good solvent | Transcutol P (mg) | 100 | 100 | 100 |
| Absorption enhancer, phospholipid | Species | PL100M | | |
| | Dose (mg) | 100 | 100 | 100 |
| Diluent | Species | Ethyl oleate | Propylene glycol lau-rate | Propylene glycol dicaprylate/di-capr ate |
| | Dose (mg) | 785 | 785 | 785 |
| Appearance (room temperature) | | yellow transparent | Orange transparent | yellow transparent |
| Permeability coefficient (cm/s) $10^{-7}$ | | $9.6 \pm 3.2$ | $2.5 \pm 0.6$ | $5.1 \pm 1.3$ |
| Nasal cavity local irritation | | Reach the standard | Reach the standard | Reach the standard |

Experimental conclusion: firstly, for phospholipid used as absorption enhancer, lecithin PL-100M and soya lecithin S75 show better absorption via nasal mucosal permeation. Secondly, for the diluent, ethyl oleate and isopropyl myristate show best absorption via nasal mucosal permeation (*see* composition 4-4 in Table 6 and composition 4-2 in Table 5), followed by propylene glycol dicaprylate/dicaprate (*see* composition 4-6 in Table 6).

Considering the preceding effects of the absorption enhancer and the diluent on the appearance, the nasal cavity local irritation and the permeability coefficient of the nasal mucosa of the presently disclosed nose-to-brain delivery formulation of the nimodipine, the presently disclosed nose-to-brain delivery formulation of the nimodipine preferably employs the lecithin or the soya lecithin as the absorption enhancer; and preferably employs one or more of the ethyl oleate, the isopropyl myristate, the propylene glycol dicaprylate/dicaprate as the diluent.

Embodiment 5:

The nose-to-brain delivery formulation of nimodipine in the present disclosure is prepared according to the composition shown in Table 7, the specific preparation method is as follows: a formulated amount of the nimodipine and a good solvent Transcutol P are weighed into a glass vial and dissolved by sonication; a corresponding amount of an absorption enhancer (a variety of phospholipid) is then added according to specific components of each composition and is dissolved by shaking and sonication; and the formulated amount of the diluent ethyl oleate is finally added until a total mass of each composition reaches 1 g and mixed by rotation for 30 minutes to be dispersed until homogeneous to obtain the following composition.

Table 7

| Composition number | Dose of Lecithin PL-100M | | | Dose of Transcutol P | | | | Low concentration | Soya lecithin replacement | | | |
|---|---|---|---|---|---|---|---|---|---|---|---|---|
| | Composition 5-1 | Composition 5-2 | Composition 5-3 | Composition 5-4 | Composition 5-5 | Composition 5-6 | Composition 5-7 | Composition 5-8 | Composition 5-9 | Composition 5-10 | Composition 5-11 | Composition 5-12 |
| Nimodipine (mg) | 15 | 15 | 15 | 15 | 15 | 15 | 15 | 5 | 15 | 15 | 15 | 15 |
| Transcutol P (mg) | 100 | 100 | 100 | 60 | 80 | 120 | 140 | 100 | 100 | 100 | 100 | 100 |
| Lecithin PL-100M (mg) | 60 | 80 | 120 | 100 | 100 | 100 | 100 | 100 | - | - | - | - |
| Ethyl oleate | 825 | 805 | 765 | 825 | 805 | 765 | 745 | 795 | 785 | 785 | 785 | 785 |
| Purified Lecithin (mg) | - | - | - | - | - | - | - | - | 100 | - | - | - |
| Domestic soya lecithin (mg) | - | - | - | - | - | - | - | - | - | 100 | - | - |
| soya lecithin S75 (mg) | - | - | - | - | - | - | - | - | - | - | 100 | - |
| Soya lecithin S100 (mg) | - | - | - | - | - | - | - | - | - | - | - | 100 |
| Density (g/ml) | 0.8992 | 0.9007 | 0.9040 | 0.8990 | 0.8898 | 0.8911 | 0.9052 | 0.8991 | 0.9016 | 0.9064 | 0.9019 | 0.9041 |
| Permeability coefficient (cm/s) $10^{-7}$ | 4.8 ± 3.3 | 4.7 ± 2.9 | 5.5 ± 1.2 | 6.2 ± 1.8 | 4.5 ± 3.2 | 9.3 ± 2.8 | 6.8 ± 0.8 | 4.9 ± 2.0 | 7.5 ± 0.9 | 6.8 ± 1.0 | 5.7 ± 2.0 | 8.7 ± 3.6 |

As can be seen from Table 7 above, firstly, the type of phospholipid has little effect on the permeability coefficient of the nose-to-brain delivery formulation of the nimodipine of the present disclosure (*see* Table 7). Secondly, Transcutol P and phospholipid in an amount of 60 to 140 parts by weight are still able to meet the requirements of the permeability coefficient and nasal local irritation for the nose-to-brain delivery formulation (*see* Table 7). Finally, a decrease in API concentration reduces local absorption, and a high API concentration favors local absorption (*see* Formulations 5-8 in Table 7).

Comparative Embodiment 1:

The composition of the comparative embodiment shown in Tables 8 and 9, a formulated amount of nimodipine and a corresponding good solvent are weighed into a glass vial and dissolved by sonication; a corresponding amount of an absorption enhancer and/or surfactant is then added according to specific components of each composition and is dissolved by shaking and sonication; the formulated amount of the diluent is finally added until a total mass of each composition reaches 1 g and mixed by rotation for 30 minutes to be dispersed until homogeneous.

Table 8

| Composition number | | Comparative Embodiment 1-1 | Comparative Embodiment 1-2 | Comparative Embodiment 1-3 | Comparative Embodiment 1-4 |
|---|---|---|---|---|---|
| Nimodipine (mg) | | 25 | 25 | 5 | 5 |
| Good Solvent (mg) | Benzyl alcohol | 9 | 9 | / | / |
| | PEG400 | 100 | 100 | / | / |
| | Ethanol | 50 | 50 | 30 | 30 |
| | Transcutol P | / | / | 45 | 45 |
| Surfactant | Species | Tween 80 | Tween 20 | Tween 80 | Labrafil® M1944cs |
| | Dose (mg) | 5 | 25 | 1 | 40 |
| Absorption enhancer | DDM (mg) | 0.43, 2.1 | 0.43, 2.1 | 2.1 | / |
| Castor oil (mg) | | 810.57 | 790.57 | 916.9 | 884 |

Table 9

| Formulation number | | Comparative Embodiment 1-5 | Comparative Embodiment 1-6 |
|---|---|---|---|
| Nimodipine (mg) | | 25 | 15 |
| Good solvent (mg) | Benzyl alcohol | 9 | 5.4 |
| | PEG400 | 100 | 60 |
| | Ethanol | 50 | 50 |
| | Transcutol P | / | / |
| Surfactant | species | Tween 20 | Tween 20 |
| | Dose (mg) | 2.5 | 1.5 |
| Absorption enhancer | DDM (mg) | 2.1 | 2.1 |
| Castor oil (mg) | | 811.4 | 866 |

The results of the experiment are shown in Table 10 and Table 11 below:

Table 10

| Composition number | Comparative Embodiment 1-1 | Comparative Embodiment 1-2 | Comparative Embodiment 1-3 | Comparative Embodiment 1-4 |
|---|---|---|---|---|
| Nasal local irritation | Pass | Pass | Pass | Pass |

(continued)

| Composition number | Comparative Embodiment 1-1 | | Comparative Embodiment 1-2 | | Comparative Embodiment 1-3 | Comparative Embodiment 1-4 |
|---|---|---|---|---|---|---|
| permeability coefficient (cm/s)10$^{-7}$ | 0.04% DDM | 0.2% DDM | 0.04% DDM | 0.2% DDM | 0.39±0.14 | 0.46±0.22 |
| | 0.64±0.19 | 0.97±0.39 | 0.54±0.21 | 1.24±0.40 | | |

Table 11

| Formulation number | Comparative Embodiment 1-5 | Comparative Embodiment 1-6 |
|---|---|---|
| Nasal local irritation | Pass | Pass |
| permeability coefficient (cm/s) 10$^{-7}$ | 0.95±0.19 | 1.28±0.42 |

Experimental conclusion:

When alcohols are used as the good solvent in the formulation, the permeability coefficient of the nose-to-brain delivery formulation of the nimodipine, even with an addition of the surfactant or/and the absorption enhancer, remains low and does not meet the requirements for that nose-to-brain delivery formulation that enter the brain via the mucosa of the olfactory region of the nose.

Comparative Embodiment 2:

The composition shown in Tables 12 below is prepared in accordance with the conventional method by those of skill in the art and may also be prepared in accordance with an analogous method of Comparative Embodiment 1;

Tabel 12

| Composition number | | Comparative Embodiment 2-1 | Comparative Embodiment 2-2 | Comparative Embodiment 2-3 | Comparative Embodiment 2-4 | Comparative Embodiment 2-5 |
|---|---|---|---|---|---|---|
| Nimodipine (mg) | | 15 | 15 | 15 | 15 | 15 |
| Transcutol P (mg) | | 100 | 100 | 100 | 100 | 100 |
| Absorption enhancer | Species | Polyoxyethylene castor oil ELP | Tween 20 | PL100M | PL100M | PL100M |
| | Dose (mg) | 40 | 20 | 100 | 100 | 100 |
| Diluent | Spices | Castor oil | Oleic acid | Propylene glycol monocapryla te | Glyceryl mono and dicaprylocapra te | Propylene glycol monolaurate |
| | Dose (mg) | 845 | 865 | 785 | 785 | 785 |
| Permeability coefficient (cm/s) 10$^{-7}$ | | 1.27 ± 1.10 | 1.74 ± 0.30 | 1.6 ± 0.1 | coagulation occurs, permeability coefficient unmeasured | coagulation occurs, permea bility coefficient unmeasured |

Experimental conclusion: when polyoxyethylene castor oil ELP or Tween 80 are used as the absorption enhancer in the nose-to-brain delivery of the nimodipine, the permeability coefficient is difficult to meet the requirements of a nose-to-brain delivery, when lecithin is used as the absorption enhancer, the type of the diluent will affect the permeability coefficient, for example, when propylene glycol monocaprylate is used as the diluent, the permeability coefficient is only 1.6*10$^{-7}$ cm/s, when glyceryl mono and dicaprylocaprate or propylene glycol monolaurate is used, coagulation occurs, which does not meet the basic requirements of the nose-to-brain delivery formulation.

Pharmacologic Embodiment 1: comparison with a control formulation via intravenous administration

Pharmacokinetic data is assayed using compositions 5-8 in embodiment 5. The nose-to-brain delivery formulation of the nimodipine of compositions 5-8 is placed in a transnasal drug delivery device (RA-IDDS® Intelligent Integrated Nasal Drug Delivery System for Small Animals developed by the company of Applicant, as disclosed in CN202021777601.0 and CN202010148279.3), and the prescribed dose of the drug is applied to the mice transnasally, and nasal delivery is administrated under gas anesthesia for 2 minutes at a constant speed. The pharmacokinetics and distribution of the composition administered via the nasal delivery are evaluated at 2, 5, 15, 30, 60, 120, 180 minutes after the administration of the drug, and plasma, olfactory bulb, and sampling methods of cortical brain tissues are commonly used methods by those of skill in the art; and for collection of cerebrospinal fluid, a method of evacuating blood and then collecting the cerebrospinal fluid is adopted, which can try to avoid contamination of the cerebrospinal fluid by the blood, thus making the test results more accurate.

In this embodiment, ST-0 refers to Nimotop® injection (specification 50 mL/10 mg) manufactured by Bayer, and ST202-Y refers to the composition of the nose-to-brain delivery formulation of the present disclosure (compositions 5-8 prepared in Embodiment 5); the DMPK comparison data of the nose-to-brain delivery formulation via the nasal delivery versus the Nimotop® injection via intravenous administration is shown in Table 13 below:

Table 13

| Administration | Dose mg/kg | Plasma | | | |
|---|---|---|---|---|---|
| | | $C_{max}$ ng/mL | $T_{max}$ min | $AUC_{0-t}$ min·ng/mL | $t_{1/2}$ min |
| Intravenous Control ST-0 | 2 | 596.0 | 2 | 14302 | 49 |
| Nose-to-brain delivery ST202-Y | | 33.8 | 15 | 3753 | 307 |

| Administration | Dose mg/kg | Cerebrospinal fluid | | | | | |
|---|---|---|---|---|---|---|---|
| | | $C_{max}$ ng/mL | Cerebrospin al fluid/blood | $T_{max}$ min | $AUC_{0-t}$ min·ng/mL | Cerebrospin al fluid/blood | $t_{1/2}$ min |
| Intravenous Control ST-0 | 2 | 4.2 | 0.01 | 2 | 149 | 0.01 | 242 |
| Nose-to-brain delivery ST202-Y | | 3.8 | 0.11 | 180 | 229 | 0.06 | / |

| Administration | Dose mg/kg | Olfactory bulb | | | | | |
|---|---|---|---|---|---|---|---|
| | | $C_{max}$ ng/mL | Olfactory bulb/blood | $T_{max}$ min | $AUC_{0-t}$ min·ng/mL | Olfactory bulb/blood | $t_{1/2}$ min |
| Intravenous Control ST-0 | 2 | 1547.4 | 2.60 | 2 | 29834 | 2.09 | 24 |
| Nose-to-brain delivery ST202-Y | | 1322.6 | 39.13 | 5 | 55257 | 14.72 | 43 |

| Administration | Dose mg/kg | Cortical brain tissue | | | | | |
|---|---|---|---|---|---|---|---|
| | | $C_{max}$ ng/mL | Brain/blood | $T_{max}$ min | $AUC_{0-t}$ min·ng/mL | Brain/blood | $t_{1/2}$ min |
| Intravenous Control ST-0 | 2 | 1744.4 | 2.93 | 2 | 29435 | 2.06 | 36 |
| Nose-to-brain delivery ST202-Y | | 158.0 | 4.67 | 5 | 10503 | 2.80 | 84 |

When ST-0 is administered intravenously to mice, it takes 2 minutes to finish the administration, the improved composition ST202-Y of the present disclosure is administered to mice via the nasal delivery, the plasma drug concentrations in the plasma, the cerebrospinal fluid, the olfactory bulb, and the cortical brain tissues are shown in FIGS. 1-4, respectively.

As can be seen from FIG. 1, plasma drug exposure of the nasal delivery is only 1/4 of that of intravenous injection, which can reduce peripheral adverse reactions and has a higher safety profile.

The clinical use of the ST-0 Nimotop® injection is sustained intravenous administration, which is not convenient for direct comparison in animal experiments, so the cerebrospinal fluid/blood ratio, the brain/blood ratio, and DTP% (percentage of direct drug delivery) in the PK data are the most informative; and the cerebrospinal fluid/blood ratio and the brain/blood ratio via the nasal delivery are approximately 10 times higher than those via the intravenous administration as the control.

The most prominent indication for the nimodipine is subarachnoid hemorrhage, and the cerebrospinal fluid is naturally present in the subarachnoid space. As shown in FIGS. 2 and 3, the exposure of the drug in the cerebrospinal fluid via the nasal delivery is more than twice as much as that of the intravenous administration in 3 hours, which has significant advantages; the nose-to-brain delivery formulation of the present disclosure, after the nasal delivery, has a high drug concentration in the olfactory bulb, which can act as a reservoir of the drug to release the drug slowly into the cerebrospinal fluid, thus exerting effects for a long period of time; at 3 hours, a content of the ST-0 formulation via the intravenous administration in the cerebrospinal fluid has dropped to the lowest value, while the improved nose-to-brain delivery formulation ST202-Y of the present disclosure via the nasal delivery is still at a second peak at 3 hours and is still exerting therapeutic effects. As shown in FIG. 4, the ST-0 is can readily enter the brain by disrupting the blood-brain barrier via vascular irritation, resulting in a transient content increase in brain. However, on the premise that the nose-to-brain delivery formulation of the present disclosure is safe and non-irritating, it exhibits high targeting of both the cerebrospinal fluid and the cortical brain tissue.

Drugs can be absorbed into the brain via two pathways after the nasal delivery: a systemic pathway and an olfactory pathway. In order to more clearly describe direct nose-to-brain transport, i.e. brain targeting, of the nose-to-brain delivery formulation of the present disclosure, it is expressed as a DTP% value (percentage of direct drug transnasal transport);

$$DTP\% = (B_{in} - B_x) / B_{in} \times 100\%,$$

$$\frac{B_{iv}}{P_{iv}} = \frac{B_x}{P_{in}}$$

$B_x$ indicates an AUC value of part drugs entering the brain via the somatic circulation cross BBB after the nasal delivery;

$P_{iv}$, $B_{iv}$, $P_{in}$, $B_{in}$ indicate AUC values in blood and brain after the intravenous administration or the nasal delivery, respectively.

After calculation, DTP% in the cerebrospinal fluid is 83%, and DTP% in the olfactory bulb is 86%;

The nose-to-brain delivery formulation of the nimodipine in the present disclosure has a DTP% of 83% in the cerebrospinal fluid and DTP% of 86% in the olfactory bulb, and it has a strong targeting ability as it directly enters into a targeting portion such as the cerebrospinal fluid and the brain tissue without having to pass through the blood-brain barrier. The most important indication of the nimodipine is subarachnoid hemorrhage, and the cerebrospinal fluid is naturally present in the subarachnoid space, so the nose-to-brain delivery formulation of the nimodipine of the present disclosure has obvious therapeutic effects and significant advantages.

Pharmacologic Embodiment 2: comparison with a control formulation via oral administration

ST-1 is dissolved in 0.5% CMC-Na to prepare into a 1.5 mg/mL intragastric administration solution, an overall absorption and distribution test is performed with collection of plasma, cerebrospinal fluid, and brain tissues, at time points: 5, 15, 30, 60, 120, 240, 360, and 480 minutes, in parallel with 6 mice; and pharmacokinetics and distribution of the drug via the oral administration is evaluated and compared to the nose-to-brain delivery formulation ST202-Y of the present disclosure.

In this pharmacologic embodiment:

ST-1 refers to the Nimotop® tablet manufactured by Bayer;
ST202-Y-1 refers to a 5 mg/g nose-to-brain delivery formulation of compositions 5-8 prepared in Embodiment 5 of the present disclosure, with a mouse dose of 1 mg/kg, and it is administered using the RA-IDDS® Intelligent Integrated Nasal Drug Delivery System for Small Animals developed by the company of Applicant;
ST202-Y-3 refers to a 15mg/g nose-to-brain delivery formulation, with a mouse dose of 3 mg/kg, and it is administered using the RA-IDDS® Intelligent Integrated Nasal Drug Delivery System for Small Animals developed by the company of Applicant; and a preparation method of the ST202-Y-3 formulation: 1.35 g of nimodipine is weighed and is dissolved by 10.08 g of Transcutol P, 9g of soya lecithin is then added and is dissolved under stirring, 0.23g of grape essence is weighed and added to the aforementioned solution, ethyl oleate is used until a total amount of the formulation reaches 90 g, and 15mg/g of the nose-to-brain delivery formulation of the nimodipine is obtained under stirring.

The experimental results are shown in Table 14 and Table 15 below;

Tabel 14

| Administration | Dose mg/kg | Plasma | | | |
|---|---|---|---|---|---|
| | | $C_{max}$ ng/ml | $T_{max}$ min | $AUC_{0-t}$ min ng/ml | $t_{1/2}$ min |
| Oral administration control ST-1 | 10.5 | 18.9 | 30 | 1047 | 32 |
| Nose-to-brain delivery ST202-Y-1 | 1 | 25.9 | 15 | 1836 | 79 |
| Nose-to-brain delivery ST202-Y-3 | 3 | 103.7 | 15 | 10382 | 88 |

Table 15

| Administration | Dose mg/kg | Cerebrum | | | | | |
|---|---|---|---|---|---|---|---|
| | | $C_{max}$ ng/ml | Brain/ blood | $T_{max}$ min | $AUC_{0-t}$ min ng/ml | Brain/ blood | $t_{1/2}$ min |
| Oral Administration control ST-1 | 10.5 | 33.2 | 176% | 30 | 2107 | 201% | 43 |
| Nose-to-brain delivery ST202-Y-1 | 1 | 107.0 | 413% | 15 | 7610 | 414% | 211 |
| Nose-to-brain delivery ST202-Y-3 | 3 | 307.1 | 296% | 15 | 28489 | 274% | 136 |

Experimental conclusion: compared with the oral administration control group ST-1, the nose-to-brain delivery formulation of the present disclosure has obvious advantages, and the peak is reached rapidly; its required dose is only 1/10 of a dose via oral administration, its drug exposure of the plasma and cerebrum is 1.75 and 3.61 times as much as that of the oral administration control ST-1, and its peak concentration of the drug in the plasma and the cerebrum is 1.37 and 3.22 times as much as that of the oral administration control ST-1, so that the therapeutic effects are obvious;

Exposure in the brain can be more than 10 times higher than the oral administration when its dose is 1/3 of the oral administration (as shown in Figure 5);
The nose-to-brain delivery formulation of the present disclosure has a longer half-life in the plasma and the cerebrum of mice compared to the oral administration control ST-1, which reduces a number of administrations and enhances compliance.

Pharmacologic Embodiment 3: distribution of rat tissues

A method similar to that of Pharmacological Embodiment 2 is adopted, the oral administration control ST-1 employees the composition identical to that of Pharmacological Embodiment 2, and a formulation concentration of the nose-to-brain delivery formulation ST202-Y-4 in this Pharmacological Embodiment is 15 mg/g, which is prepared as follows: 0.405 kg of nimodipine and 3.20 kg of Transcutol P are added in a reactor and stirred until dissolution, 2.70 kg of egg lecithin PL-100M is then added and stirred until dissolution, 67.50 g of grape essence and 20.59 kg of ethyl oleate are then added and stirred until dissolution to obtain it; and a drug delivery equipment the same as described in Pharmacological embodiment 2 is used for administration to rats;
The experimental results are shown in Table 16 below:

Table 16

| Administration | Dose mg/kg | Plasma | | | | |
|---|---|---|---|---|---|---|
| | | $T_{max}$ min | $C_{max}$ ng/ml | $C_{120min}$ ng/ml | $AUC_{0-120min}$ min·ng/ml | $C_{120min}/C_{max}$ % |
| Oral administration control ST-1 | 6 | 15 | 3.21 | 0.27 | 182.4 | 8.41 |

(continued)

| Administration | Dose mg/kg | Plasma | | | | |
|---|---|---|---|---|---|---|
| | | $T_{max}$ min | $C_{max}$ ng/ml | $C_{120min}$ ng/ml | $AUC_{0-120min}$ min·ng/ml | $C_{120min}/C_{max}$ % |
| Nose-to-brain delivery ST202-Y-4 | 0.6 | 15 | 17.15 | 4.08 | 932.4 | 23.79 |

| Administration | Dose mg/kg | Cerebrospinal fluid (CSF) | | | | | $C_{max}$ CSF/plasma | $AUC_{0-120min}$ CSF/ plasma |
|---|---|---|---|---|---|---|---|---|
| | | $T_{max}$ min | $C_{max}$ ng/ml | $C_{120min}$ ng/ml | $AUC_{0-120min}$ min·ng/ml | $C_{120min}/C_{max}$ % | | |
| Oral administration control ST-1 | 6 | 15 | 1.50 | 0.11 | 34.2 | 7.33 | 0.47 | 0.19 |
| Nose-to-brain delivery ST202-Y-4 | 0.6 | 120 | 3.52 | 3.52 | 167.4 | 100.00 | 0.21 | 0.18 |

| Administration | Dose mg/kg | Cerebrum | | | | | $C_{max}$ cerebrum/ plasma | $AUC_{0-120min}$ cerebrum/ plasma |
|---|---|---|---|---|---|---|---|---|
| | | $T_{max}$ min | $C_{max}$ ng/g | $C_{120min}$ ng/g | $AUC_{0-120min}$ min·ng/g | $C_{120min}/C_{max}$ % | | |
| Oral control ST-1 | 6 | 15 | 3.42 | 0.49 | 235.8 | 14.33 | 1.07 | 1.29 |
| Nose-to-brain delivery ST202-Y-4 | 0.6 | 15 | 39.47 | 8.22 | 2307.0 | 20.83 | 2.30 | 2.47 |

Experimental conclusion:

The nose-to-brain delivery of the nimodipine ST202-Y-4 is administered to rats at a dose 1/10 that of the oral administration control ST-1, drug exposures of the plasma, the cerebrospinal fluid and the cerebrum are 5.11, 4.89 and 9.78 times higher than that of the oral administration control ST-1, and peak concentrations of the plasma, the cerebrospinal fluid, and the cerebrum are 5.34, 2.35, and 11.54 times higher than that of the oral administration control ST-1, respectively.

ØDistribution in rat tissues, the cerebrum > the plasma > the cerebrospinal fluid, and an exposure ratio of cerebrum/plasma of the nose-to-brain delivery of the nimodipine ST202-Y-4 is higher than that of the oral administration control ST-1 (2.47 VS 1.29), and its peak ratio is higher than that of the oral administration control ST-1 (2.30 VS 1.07).

ØAt a terminal time point of 120 minutes, the nose-to-brain delivery of the nimodipine ST202-Y-4 distributed in tissues at concentrations corresponding to 20.83%-100.00% of a peak level, whereas the oral administration control ST-1 has been drastically reduced to 7.33%-14.33% of the peak level.

Pharmacologic Embodiment 4: 14-day rat toxicology pre-test

Saline, all excipients, high-dose and low-dose nose-to-brain delivery formulations are respectively administered intranasally to four rats of each sex in each group, and validated after 14 days of administration, exemplarily, in this pharmacological embodiment:

The all excipients and the ST-202-Y-High use a component ratio shown in Table 17 below, which can be prepared by the method disclosed in Embodiment 1 in the present disclosure or can be prepared by the method commonly known to those of skill in the art;

Table 17

| Composition | All excipients | ST202-Y-High |
|---|---|---|
| Nimodipine (g) | NA* | 7.5 |
| Transcutol P (g) | 48.0 | 60 |
| Lecithin PL-100M (g) | 40.0 | 50 |
| Ethyl oleate (g) | 311.0 | 381.25 |
| Grape essence (g) | 1.0 | 1.25 |
| Total content (g) | 400 | 500.0 |

NA* represent 0and means that no nimodipine is contained.

ST202-Y-Low, a formulation concentration of the composition is 5 mg/g, the formulation is obtained by a preparation method in which 120 g of all excipients and 60 g of the formulation ST202-Y-High are weighed and mixed.

The experimental groupings are shown in Table 18 below:

Table 18

| Group | Dose (mg/kg/dose) | Dose (mg/kg/day) | Formulation concentration (mg/g) | Administration volume (mL/kg/dose) |
|---|---|---|---|---|
| Saline | -- | -- | -- | 0.56 |
| All excipients | -- | -- | -- | 0.56 |
| ST202-Y-Low | 2.5 | 5 | 5 | 0.56 |
| ST202-Y-High | 7.5 | 15 | 15 | 0.56 |

Experimental monitoring items and their results are as follows:

1. Cage-side observation:

No abnormality in appearance, behavioral activity, mental status, respiratory status, hair status and other general status of the rats are observed during the experiment.

2. Body weight gain is as shown in Table 19 below:

Table 19

| Group (n=8, male and female each sex) | Dose (mg/kg/day) | 1-14 days body weight gain | | | |
|---|---|---|---|---|---|
| | | Male | | Female | |
| Saline | -- | 97 g | - | 37 g | - |
| All excipients | -- | 90 g | -7% | 33 g | -11% |
| ST202-Y-Low | 5 | 97 g | 0% | 30 g | -18% |
| ST202-Y-High | 15 | 87 g | -11% | 35 g | -5% |

Conclusion of body weight gain: there is no abnormality in the body weight gain of the rats in each dose group.

3. Changes in organ weights are shown in Table 20 below:

Table 20

| Group (n=8, male and female each sex) | Dose (mg/kg/day) | Brain | Heart | Liver | Spleen | Lung | Kidney |
|---|---|---|---|---|---|---|---|
| Male | | | | | | | |
| All excipients | | -2% | 6% | -4% | -11% | -11% | 0 |
| ST202-Y-Low | 5 | -2% | 0% | -9% | 3% | -4% | -3% |
| ST202-Y-High | 15 | -2% | -7% | -10% | -10% | 26% | -6% |
| Female | | | | | | | |
| All excipients | | -11% | -1% | -4% | 0 | -4% | 3% |
| ST202-Y-Low | 5 | -6% | -1% | -8% | -1% | 4% | -4% |
| ST202-Y-High | 15 | -4% | 0 | -12% | -4% | 3% | -5% |

Conclusion of the changes in the organ weights: there is no abnormality in the organ weights of the rats in each dose group.

4. Nasal cavity irritation observations:

The appearance of the nose is observed by two persons for 30 minutes before and after the drug administration, recorded by photographs or videos, and expressed as scores according to the "Grading Criteria for Local Mucosal Irritation Response". An average score of 0.00-0.40 is no irritation; the average score of 0.41-1.50 is mild irritation; the average score of 1.51-2.50 is moderate irritation; and the average score of >2.51 is severe irritation.

Evaluation of mucosal irritation: the mucosal irritation response of the rats in each dose group is graded as no irritation and is specifically shown in Table 21 below.

Table 21

| Group n=8 | Irritation score | | | | Grade of mucosal irritation response | |
|---|---|---|---|---|---|---|
| | Before administration | | After administration | | | |
| | Male | Female | Male | Female | Male | Female |
| Saline | 0 | 0 | 0 | 0 | No irritation | No irritation |
| All excipients | 0 | 0 | 0.07 | 0.02 | No irritation | No irritation |
| ST202-Y-Low | 0 | 0 | 0.09 | 0.04 | No irritation | No irritation |
| ST202-Y-High | 0 | 0 | 0.18 | 0.07 | No irritation | No irritation |

5. Pathology results:

After 14 days of administration, pathological sections of the rats are taken, and the experimental results revealed that: there is no difference in an amount of drug exposure between male and female animals in this experiment, and there is no difference in case morphology between males and females; no pathological changes are found in the olfactory bulb of the brain or in other regions of the brain in all groups; no pathological changes are found in the mucosa of the turbinate region and the mucosa of the olfactory region in all groups; and in conclusion: the all excipients and the formulation did not show any toxicity to the rats.

It is obvious that the foregoing embodiments are merely examples for clarity of illustration and does not limit implementations. For those of ordinary skill in the art, other variations or changes in different forms can be made on the basis of the foregoing description. It is neither necessary nor possible to exhaust all embodiments herein. The obvious variations or changes derived therefrom still fall within the protection scope of the present disclosure.

**Claims**

1. A nose-to-brain delivery formulation of nimodipine, **characterized in that**, the nose-to-brain delivery formulation comprises the nimodipine, a good solvent, a diluent and an absorption enhancer, wherein the good solvent is diethylene glycol monoethyl ether, and the absorption enhancer is one or more of phospholipid, n-dodecyl-β-D-maltoside, cyclopentadecanolide, polyoxyl 15 hydroxystearate and oleoyl polyoxyl-6 glycerides, preferably, the absorption enhancer is one of the phospholipid, the cyclopentadecanolide, and more preferably is the phospholipid.

2. The nose-to-brain delivery formulation of the nimodipine of claim 1, **characterized in that**, the diluent is one or more selected from isopropyl myristate, ethyl oleate, medium chain triglycerides, castor oil, soybean oil, sesame oil, oleic acid, vitamin E, glyceryl monolinoleate, propylene glycol laurate or propylene glycol dicaprylate/dicaprate, preferably, one or a combination of multiple selected from the ethyl oleate, the isopropyl myristate, the propylene glycol dicaprylate/dicaprate.

3. The nose-to-brain delivery formulation of the nimodipine of claim 1 or 2, **characterized in that**, a content of the nimodipine is 4-50 parts by weight; and/or,

    a content of the good solvent is 5-300 parts by weight; and/or,
    a content of the diluent is 500-900 parts by weight; and/or,
    a content of the absorption enhancer is 20-200 parts by weight.

4. The nose-to-brain delivery formulation of the nimodipine of claim 3, **characterized in that**, the content of the nimodipine is 5-40 parts by weight, for example, 5, 10, 15, 20, 30, 40 parts by weight, most preferably, 15 parts by weight; and/or,

    the content of the good solvent is 40-200 parts by weight, for example, 40, 50, 80, 100, 120, 150, 160, 200 parts by weight, most preferably, 100 parts by weight; and/or,
    the content of the diluent is 700-850 parts by weight, for example, 725, 750, 785, 805, 850 parts by weight, most preferably, 785 parts by weight, and/or,
    the content of the absorption enhancer is 40-160 parts by weight, for example, 50, 60, 80, 100, 120, 150, 160 parts by weight, most preferably, 100 parts by weight.

5. The nose-to-brain delivery formulation of the nimodipine of claim 1, 2 or 4, **characterized in that**, the phospholipid is one of lecithin or soya lecithin, and/or,

    the lecithin is the lecithin of any model number, for example, the lecithin is lecithin E80 or lecithin PL100M; and the soya lecithin is the soya lecithin of any model number, for example, the soya lecithin is soya lecithin S75 or soya lecithin S100.

6. The nose-to-brain delivery formulation of nimodipine of claim 1, 2 or 4, **characterized in that**, the nose-to-brain delivery formulation of the nmodipine further contains aromatizer, the aromatizer is preferably grape essence, a content of essence is preferbaly 0-50 parts by weight, and more preferbaly 1-10 parts by weight; for example, 2.5 parts by weight.

7. The nose-to-brain delivery formulation of nimodipine of claim 1, 2 or 4, **characterized in that**, the nose-to-brain delivery formulation of the nimodipine comprises 5-40 parts by weight of the nimodipine, 40-200 parts by weight of the diethylene glycol monoethyl ether, 40-160 parts by weight of the phospholipid; 700-850 parts by weight of the isopropyl myristate, preferably, the phospholipid is the lecithin or the soya lecithin, wherein the lecithin is the lecithin of any model number, for example, lecithin E80 or lecithin PL100M, and the soya lecithin is the soya lecithin of any model number, for example, soya lecithin S75 or soya lecithin S100; or

    the nose-to-brain delivery formulation of the nimodipine comprises 15 parts by weight of the nimodipine, 100 parts by weight of the diethylene glycol monoethyl ether, 100 parts by weight of the phospholipid, 785 parts by weight of the isopropyl myristate, preferably, the phospholipid is the lecithin or the soya lecithin, wherein the lecithin is the lecithin of any model number, for example, lecithin E80 or lecithin PL100M, and the soya lecithin is the soya lecithin of any model number, for example, soya lecithin S75 or soya lecithin S100; or the nose-to-brain delivery formulation of the nimodipine comprises 5-40 parts by weight of the nimodipine, 40-200 parts by weight of the diethylene glycol monoethyl ether, 40-160 parts by weight of the phospholipid, 700-850 parts by weight of the propylene glycol dicaprylate/dicaprate, preferably, the phospholipid is lecithin or soya lecithin, wherein the lecithin is the lecithin of any model number, for example, lecithin E80 or lecithin PL100M, and the soya lecithin is the soya lecithin of any model number, for example, soya lecithin S75 or soya lecithin S100; or
    the nose-to-brain delivery formulation of the nimodipine comprises 15 parts by weight of the nimodipine, 100 parts by weight of the diethylene glycol monoethyl ether, 100 parts by weight of the lecithin, 785 parts by weight of the propylene glycol dicaprylate/dicaprate, wherein the lecithin is the lecithin of any model number, for example, lecithin E80 or lecithin PL100M; or

the nose-to-brain delivery formulation of the nimodipine comprises 5-40 parts by weight of the nimodipine, 40-200 parts by weight of the diethylene glycol monoethyl ether, 40-160 parts by weight of the phospholipid, 700-850 parts by weight of the ethyl oleate, preferably, the phospholipid is lecithin or soybean lecithin, wherein the lecithin is the lecithin of any model number, for example, lecithin E80 or lecithin PL100M, the soya lecithin is the soya lecithin of any model number, for example, soya lecithin S75 or soya lecithin S100; or

the nose-to-brain delivery formulation of the nimodipine comprises 5 parts by weight of the nimodipine, 100 parts by weight of the diethylene glycol monoethyl ether, 100 parts by weight of the lecithin, 795 parts by weight of the ethyl oleate, wherein the lecithin is any model number, for example, lecithin E80 or lecithin PL100M; or

the nose-to-brain delivery formulation of the nimodipine comprises 15 parts by weight of the nimodipine, 60 parts by weight of the diethylene glycol monoethyl ether, 100 parts by weight of the lecithin, 825 parts by weight of the ethyl oleate, wherein the lecithin is any model number, for example, lecithin E80 or lecithin PL100M; or

the nose-to-brain delivery formulation of the nimodipine comprises 15 parts by weight of the nimodipine, 120 parts by weight of the diethylene glycol monoethyl ether, 100 parts by weight of the lecithin, 765 parts by weight of the ethyl oleate, wherein the lecithin is any model number, for example, lecithin E80 or lecithin PL100M; or

the nose-to-brain delivery formulation of the nimodipine comprises 15 parts by weight of the nimodipine, 100 parts by weight of the diethylene glycol monoethyl ether, 100 parts by weight of the phospholipid, 785 parts by weight of the ethyl oleate, preferably, the phospholipid is lecithin or soya lecithin, wherein the lecithin is the lecithin of any model number, for example, lecithin E80 or lecithin PL100M, and the soya lecithin is the soya lecithin of any model number, for example, soya lecithin S75 or soya lecithin S100; or

the nose-to-brain delivery formulation of the nimodipine comprises 15 parts by weight of the nimodipine, 100 parts by weight of the diethylene glycol monoethyl ether, 60 parts by weight of the phospholipid, 825 parts by weight of the ethyl oleate, preferably, the phospholipid is lecithin or soya lecithin, wherein the lecithin is the lecithin of any model number, for example, lecithin E80 or lecithin PL100M, and the soya lecithin is the soya lecithin of any model number, for example, soya lecithin S75 or soya lecithin S100; or

the nose-to-brain delivery formulation of the nimodipine comprises 15 parts by weight of the nimodipine, 100 parts by weight of the diethylene glycol monoethyl ether, 120 parts by weight of the phospholipid, 765 parts by weight of the ethyl oleate, preferably, the phospholipid is lecithin or soya lecithin, wherein the lecithin is the lecithin of any model number, for example, lecithin E80 or lecithin PL100M, and the soya lecithin is the soya lecithin of any model number, for example, soya lecithin S75 or soya lecithin S100.

8. The nose-to-brain delivery formulation of the nimodipine of claim 1, 2 or 4, **characterized in that**, the nose-to-brain delivery formulation of the nimodipine acts as a drug reservoir.

9. A method for preparing a nose-to-brain delivery formulation of nimodipine, **characterized in that**, comprising, adding the nimodipine and the good solvent described in any one of claims 1-8 to a container, and dissolving; and adding the absorption enhancer described in any one of claims 1-8, and dissolving; and optionally, adding the aromatizer described in claim 6, and finally, obtaining by adding the diluent described in any one of claims 1-8 to a total mass and mixing.

10. A use of a nose-to-brain delivery formulation of nimodipine in a preparation of drug for treating cerebral ischemic diseases, cerebral vasospasm induced by subarachnoid hemorrhage, sudden deafness.

FIG. 1

FIG. 2

FIG. 3

FIG. 4

FIG. 5

## INTERNATIONAL SEARCH REPORT

| International application No. |
|---|
| **PCT/CN2024/109725** |

### A. CLASSIFICATION OF SUBJECT MATTER

A61K 31/4422(2006.01)i; A61K 9/00(2006.01)i; A61P 9/10(2006.01)i; A61P 25/00(2006.01)i

According to International Patent Classification (IPC) or to both national classification and IPC

### B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

WPABSC, CNTXT, CNABS, WPABS, DWPI, ENTXTC, Web of Science, CNKI, 百度, BAIDU, 万方, WANFANG, 必应, BING: 尼莫地平, 鼻, 脑, 递送制剂, 良溶剂, 二乙二醇单乙醚, 吸收促进剂, 磷脂, 稀释剂, 油酸乙酯, 肉豆蔻酸异丙酯, 丙二醇二辛酸癸酸酯, nimodipine, intranasal, brain, delivery preparation, solvent, diethylene glycol monoethyl ether, Transcutol P, absorption enhancer, phospholipid, diluent, ethyl oleate, isopropyl myristate, propylene glycol dioctanoate

### C. DOCUMENTS CONSIDERED TO BE RELEVANT

| Category* | Citation of document, with indication, where appropriate, of the relevant passages | Relevant to claim No. |
|---|---|---|
| Y | ZHANG, Qizhi et al. "Preparation of Nimodipine-loaded Microemulsion for Intranasal Delivery and Evaluation on the Targeting Efficiency to the Brain" *International Journal of Pharmaceutics*, Vol. 275, 31 December 2004 (2004-12-31), abstract, and page 88, table 1 | 1-10 |
| Y | CN 104706597 A (TIANJIN UNIVERSITY OF TRADITIONAL CHINESE MEDICINE) 17 June 2015 (2015-06-17) claim 1 | 1-10 |
| Y | CN 112912066 A (JIANGSU JIUXU HAITIAN PHARMACEUTICAL CO., LTD. et al.) 04 June 2021 (2021-06-04) claims 1, 5, and 6 | 1-10 |
| A | CN 1679561 A (FUDAN UNIVERSITY) 12 October 2005 (2005-10-12) claims 1-11 | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 November 2024** | **11 November 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/109725**

### C.    DOCUMENTS CONSIDERED TO BE RELEVANT

| Category* | Citation of document, with indication, where appropriate, of the relevant passages | Relevant to claim No. |
|---|---|---|
| A | CN 101618020 A (SHENYANG PHARMACEUTICAL UNIVERSITY) 06 January 2010 (2010-01-06) claims 1-9 | 1-10 |
| A | US 2020163947 A1 (NORTIC HOLDINGS INC.) 28 May 2020 (2020-05-28) claims 1-17 | 1-10 |

Form PCT/ISA/210 (second sheet) (July 2022)

## INTERNATIONAL SEARCH REPORT
### Information on patent family members

| International application No. |
| --- |
| **PCT/CN2024/109725** |

| Patent document cited in search report | | | Publication date (day/month/year) | Patent family member(s) | | | Publication date (day/month/year) |
| --- | --- | --- | --- | --- | --- | --- | --- |
| CN | 104706597 | A | 17 June 2015 | None | | | |
| CN | 112912066 | A | 04 June 2021 | WO | 2020048533 | A1 | 12 March 2020 |
| | | | | EP | 3848021 | A1 | 14 July 2021 |
| | | | | EP | 3848021 | A4 | 29 November 2023 |
| | | | | JP | 2021535932 | A | 23 December 2021 |
| | | | | US | 2021338649 | A1 | 04 November 2021 |
| CN | 1679561 | A | 12 October 2005 | None | | | |
| CN | 101618020 | A | 06 January 2010 | None | | | |
| US | 2020163947 | A1 | 28 May 2020 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202021777601
- CN 202010148279
- CN 201811046629
- CN 200510023273

**Non-patent literature cited in the description**

- **RUDREE PATHAK et al.** *Acta Pharmaceutica Sinica B*, 2014, vol. 4 (2), 151-160
- **JIANG** ; **XINGUO** ; **CUI** ; **JINGBIN**. Toxicity of drugs on nasal mucocilia and the method of its evaluation [J].. *Acta Pharmaceutica Sinica*, 1995, vol. 30, 848-53
- **LI, XINFANG** ; **LI, XIANGUI** ; **MA, ZHIQIANG et al.** An in situgel system for nasal delivery of menthol:preparation and safety evaluation [J].. *Journal of Pharmaceutical Practice*, 2017, vol. 3 (4), 321-4